# EUROPEAN PATENT SPECIFICATION

(11) **EP 3 347 709 B1**
(45) Date of publication and mention of the grant of the patent: **01.11.2023**
(21) Application number: 16763544.0
(22) Date of filing: 13.09.2016
(51) Int. Cl.: G01N 27/327, G01N 33/487, G01N 33/02

(54) **HANDHELD APPARATUS, DISPOSABLE TEST UNIT AND HANDHELD TEST METER FOR TESTING A SAMPLE OF PREPARED FOOD FOR ALLERGENS AND/OR FOOD INTOLERANCE INGREDIENTS**
TRAGBARE VORRICHTUNG, EINWEGTESTEINHEIT UND TRAGBARES TESTMESSGERÄT ZUM TESTEN EINER PROBE EINES ZUBEREITETEN LEBENSMITTELS AUF ALLERGENE UND/ODER LEBENSMITTELUNVERTRÄGLICHKEITSBESTANDTEILE
APPAREIL PORTATIF, UNITÉ DE TEST JETABLE ET APPAREIL DE MESURE DE TEST PORTATIF POUR TESTER UN ÉCHANTILLON D'ALIMENT PRÉPARÉ POUR ALLERGÈNES ET/OU INGRÉDIENTS D'INTOLÉRANCE ALIMENTAIRE

(30) Priority: 13.09.2015 DK 201500540
(43) Date of publication of application: 18.07.2018
(73) Proprietor: Pro-ino Development APS, 7400 Herning (DK)
(72) Inventor: HANSEN, Jens, 9330 Dronninglund (DK)
(74) Representative: Larsen & Birkeholm A/S
(86) International application number: PCT/EP2016/071544
(87) International publication number: WO 2017/042397

(56) References cited:
- WO-A2-2015/017442
- DE-A1-102006 045 952
- US-A1- 2002 084 184
- US-A1- 2009 223 287
- US-A1- 2010 015 006
- US-A1- 2011 102 152
- US-A1- 2014 295 406
- US-A1- 2015 083 609

## Description

### Technical field of the invention

The present invention relates to handheld apparatuses, electrochemical test units and handheld test meters for testing a sample of prepared food for allergens and/or food intolerance ingredients.

### Background of the invention

The medical sequelae of food allergies and food intolerances can range from a mild reaction, such as nasal congestion to anaphylactic shock, and potential death. Non-limiting examples of foods which may elicit an allergic reaction or a food intolerance-associated reaction include nuts such as peanuts and tree nuts (such as almonds, cashews, hazels walnuts and pecans), wheat (gluten) and in particular gluten, dairy (e.g. milk (lactose)), eggs, fish and shellfish.

People with severe food allergies/intolerance typically rely on trust and reputation when it comes to prepared food and possible allergens and/or food intolerance ingredients therein. If available, product labels are carefully scrutinized, and prepared food products that are labelled in a way that meets the consumer's confidence are selected.

In cases where a person with food allergy visits a restaurant, the person must rely on the report of staff about whether the food is prepared in contact with an allergen and/or food intolerance ingredient.

It is therefore desirable to provide an easy-to-use method that can identify the presence of an allergen and/or food intolerance ingredients (e.g., lactose) in prepared food to provide additional confidence to someone suffering from severe food allergies and intolerances, in a context, where the source of food is not trusted.

Self-testing of prepared food in restaurants, at home, or at parties for allergens and/or food intolerance ingredients will aid those suffering from allergies to live more safely. Often, it may be necessary to test for various allergens and/or food intolerance ingredients. Hence, there is a need for a system capable of handling multiple types of tests.

US20110102152 discloses a double side identification notation-containing test strip and a test instrument therefor. The test strip comprises a substrate, having a first surface and a second surface, and at least a test area and an identification area provided thereon, hereby forming a double side identification notation-containing test strip. In said double side identification notation-containing test strip, a test area and an identification area are disposed respectively on different surfaces, thus increasing the area of said identification area and sets of digital identification electrodes, and when it is operated in cooperation with a resistor, it generates a plurality sets of analog identification signals.

DE 10 2006 045 952 A1 discloses a biosensor with a test strip and a meter. A passive RFID tag is arranged on the test strip. A RFID-reader is arranged inside the meter for receiving of the radio frequency signals of the RFID-chip. The received RF signals are sent to a micro processing unit for releasing the calibration operation at the meter.

Similarly US 2009/223287 A1 discloses a bio-monitoring system where a RFID tag is embedded in a test strip and a RFID reader is embedded in a test meter.

### Summary of the invention

A first aspect relates to a handheld apparatus as defined in claim 1. A second aspect relates to a disposable electrochemical test unit as defined in claim 8.

A third aspect relates to a handheld test meter as defined in claim 13.

### Detailed description of the invention

A first aspect relates to a handheld apparatus as defined in claim 1.

Disclosed herein is a handheld apparatus for testing a sample of prepared food for allergens and/or food intolerance ingredients. The apparatus comprises a handheld test meter adapted for receiving a disposable electrochemical test unit.

The handheld apparatus also comprises a disposable electrochemical test unit adapted for electrochemical sensing of an allergen and/or food intolerance ingredient. The handheld test meter works in conjunction with the disposable electrochemical test unit that comprises at least two electrodes (working and counter electrodes) disposed within the receptacle.

The handheld test meter contains the electronics for applying the potential between the working and counter electrodes in the disposable electrochemical test unit, observing the current, and generating a determination of allergen and/or food intolerance ingredient and conveying it to the user. An indication of the presence or absence of detectable amounts of allergen and/or food intolerance ingredient in the sample is generated based on the observed current. Detection of allergen and/or food intolerance ingredient in a sample of prepared food refers to the qualitative, semi-quantitative or quantitative determination of whether an allergen and/or food intolerance ingredient is present in a sample at detectable levels. Qualitative analysis provides merely a yes/no answer as to whether there is detection. Semi-quantitative provides an indication of amount, but with high granularity, for example as presented through a series of lights where the number of lights illuminated indicates the range into which a value falls. Quantitative analysis provides a numerical value for the amount of allergen and/or food intolerance ingredient in the measured sample.

In order to handle different types of electrochemical tests, the handheld test meter is configured to adjust to such situations by running a specific application for each specific electrochemical test. The information about which specific electrochemical test application there is to be run is provided in the disposable electrochemical test unit by an electronic identification tag embedded therein.

The electronic identification tag is a passive RFID tag (Radio Frequency Identification), preferably a passive NFC (Near Field Communication) tag. Passive RFID tags, such as a passive NFC tag, do not have their own power source. Instead, they are powered by the electromagnetic energy transmitted from the RFID reader. Because the radio waves must be strong enough to power the tags, passive RFID tags have a read range from near contact and up to 25 meters. For the present application, it is preferred that the RFID tags are configured to have a read range from direct contact (0 mm) up to a few millimeters, such as within the range of 0-2 mm. This is to avoid confusion when several disposable electrochemical test units are in close proximity of the handheld apparatus. The NFC technology is suitable for such use.

In one or more embodiments, the RFID tag is configured to have a read range within the range of 0-20 mm, such as within the range of 1-15 mm, preferably within the range of 0-5 mm.

In order to secure the correct position between the electronic identification tag and its reader, the electronic identification tag is embedded in the connector.

The handheld test meter comprises an insertion hole comprising a clamping section; and the connector comprises an engagement groove adapted to be engaged with an engagement member in the clamping section. In one or more embodiments, the engagement member is an engagement click member. The sound of a "click" reassures the user that the disposable electrochemical test unit is correctly positioned within the insertion hole of the handheld test meter.

The electronic identification tag is embedded in the engagement groove, and the engagement member comprises an electronic identification tag reader.

In one or more embodiments, the handheld test meter comprises an insertion hole comprising a clamping section; and the connector comprises an engagement member adapted to be engaged with an engagement groove in the clamping section. In one or more embodiments, the engagement member is an engagement click member. The sound of a "click" reassures the user that the disposable electrochemical test unit is correctly positioned within the insertion hole of the handheld test meter.

In one or more embodiments, the electronic identification tag is embedded in the engagement groove, and the engagement member comprises an electronic identification tag reader.

Because it can be very important to know the presence and/or concentration of allergen and/or food intolerance ingredient in prepared food, particularly for people with food allergy or intolerance, a handheld apparatus has been developed by the inventor to enable the average person to sample and test a prepared food product for determining its allergen and/or food intolerance ingredient concentration at any given time. In one or more embodiments, the disposable electrochemical test unit comprises a receptacle (sample receiving chamber) for a sample of prepared food.

In one or more embodiments, the disposable electrochemical test unit also comprises a reagent composition disposed in the receptacle. The reagent composition may in one or more embodiments comprise an active redox enzyme effective to oxidize or reduce the allergen and/or food intolerance ingredient. A "redox enzyme" refers to an enzyme that catalyzes oxidation or reduction of the allergen and/or food intolerance ingredient to be determined. Exemplary redox enzymes are oxidases such as glucose oxidase, and dehydrogenases such as glucose dehydrogenase or lactate dehydrogenase. The redox enzyme has an active form prior to interaction with the allergen and/or food intolerance ingredient, and an inactive form. In the case of an oxidase, for example, the active form is oxidized and the inactive form is reduced. As will be apparent, the specific enzyme is selected based on its specificity for the allergen and/or food intolerance ingredient to be analyzed. Non-limiting examples of suitable enzymes include glucose oxidase, fructose dehydrogenase, glucose dehydrogenase, alcohol oxidase, lactate oxidase, cholesterol oxidase, xanthine oxidase, and amino acid oxidases.

In one or more embodiments, the reagent composition disposed in the receptacle comprises an electron transfer reagent. An "electron transfer reagent" refers to a compound other than a redox enzyme that receives and donates electrons to and/or from another chemical species or to and/or from an electrode.

In one or more embodiments, the reagent composition disposed in the receptacle comprises a mediator compound. A "mediator compound" refers to an electron transfer reagent that in use reacts with the inactive enzyme to regenerate active enzyme. The mediator compound may also transfer electrons to or from the electrodes although this is not required.

In one or more embodiments, the reagent composition disposed in the receptacle comprises a shuttle compound. A "shuttle compound" refers to an electron transfer reagent that in use transfers electrons to and from the electrodes. In some embodiments of the invention, the shuttle is reduced by transfer of an electron from one electrode and oxidized by transfer of an electron to the other electrode. In other embodiments, the shuttle can also be reduced (or oxidized) by transfer of an electron from the reduced mediator (or to the oxidized mediator). In this instance, in one preferred embodiment, the mediator itself does not react at the electrodes.

In some embodiments, the mediator compound does not transfer electrons directly to or from the shuttle compound. In other embodiments, electron transfer between mediator and shuttle in solution can happen and in other embodiments electron transfer between mediator and shuttle is the most significant method of transferring electrons from the mediator.

The allergen and/or food intolerance ingredient current generated by the redox processes is detected by the handheld test meter, and converted into an allergen and/or food intolerance ingredient concentration reading using an algorithm that relates the test current to an allergen and/or food intolerance ingredient concentration via a simple mathematical formula known within the art of electrochemical testing.

In one or more embodiments, the disposable electrochemical test unit comprises a diluent and/or solvent reservoir in liquid communication with the receptacle. This is an advantage when the sample of prepared food is too viscous/thick for the reagent composition to mix with the sample of prepared food; or for the situation where the sample of prepared food needs to be solubilized prior to testing.

In one or more embodiments, the diluent and/or solvent reservoir is configured as a foil sealed reservoir, a squeeze to burst packet, a peel apart welded compartment, or a squeeze to burst line sealed compartment.

In one or more embodiments, the disposable electrochemical test unit comprises a receptacle for a sample of prepared food, and a diluent and/or solvent reservoir in liquid communication with the receptacle.

In one or more embodiments, the receptacle is configured as a scoop, corer, spoon or pipette. This is an advantage for better sampling of the prepared food.

A second aspect relates to a disposable electrochemical test unit as defined in claim 8.

A third aspect relates to a handheld test meter as defined in claim 13.

It should be noted that embodiments and features described in the context of one of the aspects of the present invention also apply to the other aspects of the invention.

## Claims

1. A handheld apparatus configured for testing a sample of prepared food for multiple types of allergens and/or food intolerance ingredients; the apparatus comprising:
- a handheld test meter adapted for receiving a disposable electrochemical test unit; and
- a disposable electrochemical test unit configured for electrochemical sensing of an allergen and/or food intolerance ingredient;
wherein said disposable electrochemical test unit comprises a connector configured for connecting with the handheld test meter;
wherein an electronic identification tag is embedded in said disposable electrochemical test unit, said electronic identification tag comprising information on the type of disposable electrochemical test unit; wherein said electronic identification tag is a passive RFID tag;
wherein said handheld test meter is configured to read said electronic identification tag, and in response to its information on the type of disposable electrochemical test unit, configured to detect and process the electrochemical signal generated by the disposable electrochemical test unit during use to provide an indication of presence and/or amount of allergen and/or food intolerance ingredient to the user; **characterized in that** said handheld test meter comprises an insertion hole comprising a clamping section with an engagement member; wherein said connector comprises an engagement groove adapted to be engaged with the engagement member in the clamping section, wherein the electronic identification tag is embedded in the engagement groove, wherein the engagement member comprises an electronic identification tag reader.

2. The handheld apparatus according to claim 1, wherein said electronic identification tag is a passive Near Field Communication tag.

3. The handheld apparatus according to any one of the claims 1-2, wherein said tag is configured to have a read range within the range of 0-20 mm, preferably within the range of 0-2 mm.

4. The handheld apparatus according to any one of the claims 1-3, wherein said disposable electrochemical test unit comprises a receptacle configured as a sample receiving chamber.

5. The handheld apparatus according to claim 4, wherein said disposable electrochemical test unit comprises a reagent composition disposed in said receptacle and comprises an active redox enzyme effective to oxidize or reduce the allergen and/or food intolerance ingredient.

6. The handheld apparatus according to claim 5, wherein said redox enzyme is selected from the group consisting of glucose oxidase, fructose dehydrogenase, glucose dehydrogenase, alcohol oxidase, lactate oxidase, cholesterol oxidase, xanthine oxidase, and amino acid oxidases.

7. A handheld apparatus according to any one of the claims 4-6, wherein said disposable electrochemical test unit comprises a receptacle for a sample of prepared food, and a diluent and/or solvent reservoir in liquid communication with the receptacle.

8. A disposable electrochemical test unit adapted for electrochemical sensing of an allergen and/or food intolerance ingredient; said disposable electrochemical test unit being configured for electrochemical sensing of an allergen and/or food intolerance ingredient;
wherein said disposable electrochemical test unit comprises a connector configured for connecting with a handheld test meter;
wherein an electronic identification tag is embedded in said disposable electrochemical test unit, said electronic identification tag comprising information on the type of disposable electrochemical test unit;
wherein said electronic identification tag is a passive RFID tag;
**characterized in that** said connector comprises an engagement groove adapted to be engaged with an engagement member in said clamping section of said handheld test meter, and wherein said electronic identification tag is embedded in said engagement groove.

9. The disposable electrochemical test unit according to claim 8, wherein said electronic identification tag is a passive Near Field Communication tag.

10. The disposable electrochemical test unit according to any one of the claims 8-9, wherein said tag is configured to have a read range within the range of 0-20 mm, preferably within the range of 0-2 mm.

11. The disposable electrochemical test unit according to any one of the claims 8-10, further comprising a receptacle configured as a sample receiving chamber.

12. The disposable electrochemical test unit according to claim 11, further comprising a reagent composition disposed in said receptacle, said reagent composition comprising an active redox enzyme effective to oxidize or reduce said allergen and/or food intolerance ingredient.

13. A handheld test meter adapted for receiving a disposable electrochemical test unit; wherein the handheld test meter is configured to read an electronic identification tag being a passive RFID tag;
wherein said passive RFID tag is embedded in a disposable
electrochemical test unit, and in response to its information on the type of disposable electrochemical test unit, configured to detect and process the electrochemical signal generated by the disposable electrochemical test unit during use to provide an indication of presence and/or amount of allergen and/or food intolerance ingredient to the user;
**characterized in that** said handheld test meter comprises an insertion hole comprising a clamping section comprising an engagement member adapted for receiving an engagement groove of a disposable electrochemical test unit, and wherein said engagement member comprises an electronic identification tag reader adapted for reading the passive RFID tag.

14. The handheld test meter according to claim 13, wherein said electronic identification tag reader is adapted for reading a passive Near Field Communication tag.

15. Use of the handheld apparatus according to any one of the claims 1-7 for testing a sample of prepared food for multiple types of allergens and/or food intolerance ingredients.

16. Use of the disposable electrochemical test unit according to any one of the claims 8-12 for testing a sample of prepared food for multiple types of allergens and/or food intolerance ingredients.

17. Use of the handheld test meter according to any one of the claims 13-14 for testing a sample of prepared food for multiple types of allergens and/or food intolerance ingredients.

## Patentansprüche

1. Tragbare Vorrichtung, die dazu konfiguriert ist, eine Probe eines zubereiteten Lebensmittels auf mehrere Typen von Allergenen und/oder Lebensmittelunverträglichkeiten zu testen; wobei die Vorrichtung umfasst:
- ein tragbares Testmessgerät, das zum Aufnehmen einer elektrochemischen Einwegtesteinheit geeignet ist; und
- eine elektrochemische Einwegtesteinheit, die für die elektrochemische Erkennung eines Allergens und/oder eines Nahrungsmittelunverträglichkeitsbestandteils konfiguriert ist;
wobei die elektrochemische Einwegtesteinheit einen Anschluss umfasst, der zum Verbinden mit dem tragbaren Testmessgerät konfiguriert ist;
wobei ein elektronisches Identifikationsetikett in die elektrochemische Einwegtesteinheit eingebettet ist, wobei das elektronische Identifikationsetikett Informationen über den Typ der elektrochemischen Einwegtesteinheit umfasst; wobei das elektronische Identifikationsetikett ein passives RFID-Etikett ist;
wobei das tragbare Testmessgerät dazu konfiguriert ist, das elektronische Identifikationsetikett zu lesen, und als Reaktion auf seine Informationen über den Typ der elektrochemischen Einwegtesteinheit dazu konfiguriert ist, das elektrochemische Signal, das von der elektrochemischen Einwegtesteinheit während der Verwendung erzeugt wird, zu detektieren und zu verarbeiten, um dem Benutzer eine Anzeige über das Vorhandensein und/oder die Menge des Allergens und/oder des Nahrungsmittelunverträglichkeitsbestandteils bereitzustellen;
**dadurch gekennzeichnet, dass** das tragbare Testmessgerät ein Einführungsloch umfasst, das einen Klemmabschnitt mit einem Eingriffselement umfasst; wobei der Verbinder eine Eingriffsnut umfasst, die angepasst ist, um mit dem Eingriffselement in dem Klemmabschnitt in Eingriff gebracht zu werden, wobei das elektronische Identifikationsetikett in die Eingriffsnut eingebettet ist, wobei das Eingriffselement einen elektronischen Identifikationsetikett-Leser umfasst.

2. Tragbare Vorrichtung nach Anspruch 1, wobei das elektronische Identifikationsetikett ein passives Nahfeldkommunikationsetikett ist.

3. Tragbare Vorrichtung nach einem der Ansprüche 1-2, wobei das Etikett dazu konfiguriert ist, einen Lesebereich innerhalb des Bereichs von 0-20 mm, vorzugsweise innerhalb des Bereichs von 0-2 mm, aufzuweisen.

4. Tragbare Vorrichtung nach einem der Ansprüche 1-3, wobei die elektrochemische Einwegtesteinheit ein Gefäß umfasst, das als Probenaufnahmekammer konfiguriert ist.

5. Tragbare Vorrichtung nach Anspruch 4, wobei die elektrochemische Einwegtesteinheit eine Reagenzzusammensetzung umfasst, die in dem Behälter angeordnet ist und ein aktives Redoxenzym umfasst, das wirksam ist, um das Allergen und/oder den Nahrungsmittelunverträglichkeitsbestandteil zu oxidieren oder zu reduzieren.

6. Tragbare Vorrichtung nach Anspruch 5, wobei das Redoxenzym ausgewählt ist aus der Gruppe bestehend aus Glucoseoxidase, Fructosedehydrogenase, Glucosedehydrogenase, Alkoholoxidase, Lactatoxidase, Cholesterinoxidase, Xanthinoxidase und Aminosäureoxidasen.

7. Tragbare Vorrichtung nach einem der Ansprüche 4-6, wobei die elektrochemische Einwegtesteinheit einen Behälter für eine Probe eines zubereiteten Lebensmittels und einen Verdünnungsmittel- und/oder Lösungsmittelbehälter in Flüssigkeitsverbindung mit dem Behälter umfasst.

8. Elektrochemische Einwegtesteinheit, die zum elektrochemischen Nachweis eines Allergens und/oder eines Nahrungsmittelunverträglichkeitsbestandteils geeignet ist, wobei die elektrochemische Einwegtesteinheit zum elektrochemischen Nachweis eines Allergens und/oder eines Nahrungsmittelunverträglichkeitsbestandteils konfiguriert ist;
wobei die elektrochemische Einwegtesteinheit einen Anschluss umfasst, der zum Verbinden mit einem tragbaren Testmessgerät konfiguriert ist;
wobei ein elektronisches Identifikationsetikett in die elektrochemische Einwegtesteinheit eingebettet ist, wobei das elektronische Identifikationsetikett Informationen über den Typ der elektrochemischen Einwegtesteinheit umfasst; wobei das elektronische Identifikationsetikett ein passives RFID-Etikett ist;
**dadurch gekennzeichnet, dass** der Verbinder eine Eingriffsnut umfasst, die mit einem Eingriffselement in dem Klemmabschnitt des tragbaren Testmessgeräts in Eingriff gebracht werden kann, und wobei das elektronische Identifikationsetikett in die Eingriffsnut eingebettet ist.

9. Elektrochemische Einwegtesteinheit nach Anspruch 8, wobei das elektronische Identifikationsetikett ein passives Nahfeldkommunikationsetikett ist.

10. Elektrochemische Einwegtesteinheit nach einem der Ansprüche 8-9, wobei das Etikett dazu konfiguriert ist, einen Lesebereich innerhalb des Bereichs von 0-20 mm, vorzugsweise innerhalb des Bereichs von 0-2 mm, aufzuweisen.

11. Elektrochemische Einwegtesteinheit nach einem der Ansprüche 8-10, ferner umfassend ein Gefäß, das als Probenaufnahmekammer konfiguriert ist.

12. Elektrochemische Einwegtesteinheit nach Anspruch 11, ferner umfassend eine Reagenzzusammensetzung, die in dem Behälter angeordnet ist, wobei die Reagenzzusammensetzung ein aktives Redoxenzym umfasst, das wirksam ist, um das Allergen und/oder den Nahrungsmittelunverträglichkeitsbestandteil zu oxidieren oder zu reduzieren.

13. Tragbares Testmessgerät, das zum Aufnehmen einer elektrochemischen Einwegtesteinheit geeignet ist; wobei das tragbare Testmessgerät dazu konfiguriert ist, ein elektronisches Identifikationsetikett zu lesen, bei dem es sich um ein passives RFID-Etikett handelt; wobei das passive RFID-Etikett in eine elektrochemische Einwegtesteinheit eingebettet ist und als Reaktion auf seine Informationen über den Typ der elektrochemischen Einwegtesteinheit dazu konfiguriert ist, das von der elektrochemischen Einwegtesteinheit während des Gebrauchs erzeugte elektrochemische Signal zu detektieren und zu verarbeiten, um dem Benutzer eine Anzeige über das Vorhandensein und/oder die Menge eines Allergen- und/oder Nahrungsmittelunverträglichkeitsbestandteils bereitzustellen;
**dadurch gekennzeichnet, dass** das tragbare Testmessgerät ein Einführungsloch umfasst, das einen Klemmabschnitt aufweist, der ein Eingriffselement umfasst, das zum Aufnehmen einer Eingriffsnut einer elektrochemischen Einwegtesteinheit geeignet ist, und wobei das Eingriffselement ein elektronisches Identifikationsetikett-Lesegerät umfasst, das zum Lesen des passiven RFID-Tags geeignet ist.

14. Tragbares Testmessgerät nach Anspruch 13, wobei das elektronische Identifikationsetikett-Lesegerät zum Lesen eines passiven Nahfeldkommunikationsetiketts geeignet ist.

15. Verwendung der tragbaren Vorrichtung nach einem der Ansprüche 1-7 zum Testen einer Probe eines zubereiteten Lebensmittels auf mehrere Typen von Allergenen und/oder Lebensmittelunverträglichkeitsbestandteilen.

16. Verwendung der elektrochemischen Einwegtesteinheit nach einem der Ansprüche 8-12 zum Testen einer Probe eines zubereiteten Lebensmittels auf mehrere Typen von Allergenen und/oder Lebensmittelunverträglichkeitsbestandteilen.

17. Verwendung des tragbaren Testmessgeräts nach einem der Ansprüche 13-14 zum Testen einer Probe eines zubereiteten Lebensmittels auf mehrere Typen von Allergenen und/oder Lebensmittelunverträglichkeitsbestandteilen.

## Revendications

1. Appareil portatif configuré pour tester un échantillon d'aliment préparé pour plusieurs types d'allergènes et/ou d'ingrédients d'intolérance alimentaire ; l'appareil comprenant :
- un appareil de mesure de test portatif adapté pour recevoir une unité de test électrochimique jetable ; et
- une unité de test électrochimique jetable configurée pour la détection électrochimique d'un allergène et/ou d'un ingrédient d'intolérance alimentaire ;
dans lequel ladite unité de test électrochimique jetable comprend un connecteur configuré pour se connecter à l'appareil de mesure de test portatif ;
dans lequel une étiquette d'identification électronique est intégrée dans ladite unité de test électrochimique jetable, ladite étiquette d'identification électronique comprenant des informations sur le type d'unité de test électrochimique jetable ; dans lequel ladite étiquette d'identification électronique est une étiquette RFID passive ;
dans lequel ledit appareil de mesure de test portatif est configuré pour lire ladite étiquette d'identification électronique et, en réponse à ses informations sur le type d'unité de test électrochimique jetable, configuré pour détecter et traiter le signal électrochimique généré par l'unité de test électrochimique jetable pendant l'utilisation pour fournir une indication de la présence et/ou de la quantité d'allergène et/ou d'ingrédient d'intolérance alimentaire pour l'utilisateur ; **caractérisé en ce que** ledit appareil de mesure de test portatif comprend un trou d'insertion comprenant une section de serrage avec un élément de mise en prise ; dans lequel ledit connecteur comprend une rainure de mise en prise adaptée pour être en prise avec l'élément de mise en prise dans la section de serrage, dans lequel l'étiquette d'identification électronique est intégrée dans la rainure de mise en prise, dans lequel l'élément de mise en prise comprend un lecteur d'étiquette d'identification électronique.

2. Appareil portatif selon la revendication 1, dans lequel ladite étiquette d'identification électronique est une étiquette de communication en champ proche passive.

3. Appareil portatif selon l'une quelconque des revendications 1 et 2, dans lequel ladite étiquette est configurée pour avoir une plage de lecture comprise entre 0 et 20 mm, de préférence comprise entre 0 et 2 mm.

4. Appareil portatif selon l'une quelconque des revendications 1 à 3, dans lequel ladite unité de test électrochimique jetable comprend un réceptacle configuré comme une chambre de réception d'échantillon.

5. Appareil portatif selon la revendication 4, dans lequel ladite unité de test électrochimique jetable comprend une composition réactive disposée dans ledit réceptacle et comprend une enzyme redox active efficace pour oxyder ou réduire l'allergène et/ou l'ingrédient d'intolérance alimentaire.

6. Appareil portatif selon la revendication 5, dans lequel ladite enzyme redox est choisie dans le groupe constitué de la glucose oxydase, de la fructose déshydrogénase, de la glucose déshydrogénase, de l'alcool oxydase, de la lactate oxydase, de la cholestérol oxydase, de la xanthine oxydase et des oxydases d'acides aminés.

7. Appareil portatif selon l'une quelconque des revendications 4 à 6, dans lequel ladite unité de test électrochimique jetable comprend un réceptacle pour un échantillon d'aliment préparé, et un réservoir de diluant et/ou de solvant en communication liquide avec le réceptacle.

8. Unité de test électrochimique jetable adaptée à la détection électrochimique d'un allergène et/ou d'un ingrédient d'intolérance alimentaire ; ladite unité de test électrochimique jetable étant configurée pour la détection électrochimique d'un allergène et/ou d'un ingrédient d'intolérance alimentaire ;
dans laquelle ladite unité de test électrochimique jetable comprend un connecteur configuré pour se connecter à un appareil de mesure de test portatif ;
dans laquelle une étiquette d'identification électronique est intégrée dans ladite unité de test électrochimique jetable, ladite étiquette d'identification électronique comprenant des informations sur le type d'unité de test électrochimique jetable ; dans laquelle ladite étiquette d'identification électronique est une étiquette RFID passive ;
**caractérisée en ce que** ledit connecteur comprend une rainure de mise en prise adaptée pour être en prise avec un élément de mise en prise dans ladite section de serrage dudit appareil de mesure de test portatif, et dans laquelle ladite étiquette d'identification électronique est intégrée dans ladite rainure de mise en prise.

9. Unité de test électrochimique jetable selon la revendication 8, dans laquelle ladite étiquette d'identification électronique est une étiquette de communication en champ proche passive.

10. Unité de test électrochimique jetable selon l'une quelconque des revendications 8 et 9, dans laquelle ladite étiquette est configurée pour avoir une plage de lecture comprise entre 0 et 20 mm, de préférence comprise entre 0 et 2 mm.

11. Unité de test électrochimique jetable selon l'une quelconque des revendications 8 à 10, comprenant également un réceptacle configuré comme une chambre de réception d'échantillon.

12. Unité de test électrochimique jetable selon la revendication 11, comprenant également une composition réactive disposée dans ledit réceptacle, ladite composition réactive comprenant une enzyme redox active efficace pour oxyder ou réduire ledit allergène et/ou ingrédient d'intolérance alimentaire.

13. Appareil de mesure de test portatif adapté pour recevoir une unité de test électrochimique jetable ; dans lequel l'appareil de mesure de test portatif est configuré pour lire une étiquette d'identification électronique qui est une étiquette RFID passive ; dans lequel ladite étiquette RFID passive est intégrée dans une unité de test électrochimique jetable et, en réponse à ses informations sur le type d'unité de test électrochimique jetable, configurée pour détecter et traiter le signal électrochimique généré par l'unité de test électrochimique jetable pendant l'utilisation pour fournir une indication de la présence et/ou de la quantité d'allergène et/ou d'ingrédient d'intolérance alimentaire pour l'utilisateur ;
**caractérisé en ce que** ledit appareil de mesure de test portatif comprend un trou d'insertion comprenant une section de serrage comprenant un élément de mise en prise adapté pour recevoir une rainure de mise en prise d'une unité de test électrochimique jetable, et dans lequel ledit élément de mise en prise comprend un lecteur d'étiquette d'identification électronique adapté pour lire l'étiquette RFID passive.

14. Appareil de mesure de test portatif selon la revendication 13, dans lequel ledit lecteur d'étiquette d'identification électronique est adapté pour lire une étiquette de communication en champ proche passive.

15. Utilisation de l'appareil portatif selon l'une quelconque des revendications 1 à 7 pour tester un échantillon d'aliment préparé pour plusieurs types d'allergènes et/ou d'ingrédients d'intolérance alimentaire.

16. Utilisation de l'unité de test électrochimique jetable selon l'une quelconque des revendications 8 à 12 pour tester un échantillon d'aliment préparé pour plusieurs types d'allergènes et/ou d'ingrédients d'intolérance alimentaire.

17. Utilisation de l'appareil de mesure de test portatif selon l'une quelconque des revendications 13 et 14 pour tester un échantillon d'aliment préparé pour plusieurs types d'allergènes et/ou d'ingrédients d'intolérance alimentaire.
